# EUROPEAN PATENT SPECIFICATION

(11) **EP 3 965 687 B1**
(45) Date of publication and mention of the grant of the patent: **18.10.2023**
(21) Application number: 20721268.9
(22) Date of filing: 30.04.2020
(51) Int. Cl.: A61B 90/00, A61N 5/10

(54) **PATIENT MARKER**
PATIENTENMARKIERER
MARQUEUR DE PATIENT

(30) Priority: 06.05.2019 EP 19172740
(43) Date of publication of application: 16.03.2022
(73) Proprietor: Koninklijke Philips N.V., 5656 AG Eindhoven (NL)
(72) Inventor: VOGTMEIER, Gereon, 5656 AE Eindhoven (NL); WEISS, Steffen, 5656 AE Eindhoven (NL); LEUSSLER, Christoph, Günther, 5656 AE Eindhoven (NL); VUPPALA, Sunil, Kumar, 5656 AE Eindhoven (NL); HELLE, Michael, Günter, 5656 AE Eindhoven (NL); SISODIA, Rajendra, Singh, 5656 AE Eindhoven (NL)
(74) Representative: Philips Intellectual Property & Standards
(86) International application number: PCT/EP2020/062157
(87) International publication number: WO 2020/225134

(56) References cited:
- EP-A2- 2 101 670
- EP-B1- 2 101 670
- US-A1- 2014 270 067
- US-A1- 2018 098 820
- US-A1- 2019 008 412
- US-B2- 9 786 420

## Description

### FIELD OF THE INVENTION

The present invention relates to a patient marker, an imaging system, and a method of imaging a patient with an imaging system.

### BACKGROUND OF THE INVENTION

US2014/270067A1 relates to a radiographic marker. The marker includes a first portion of radiolucent material comprising a first radiopaque pattern. The first portion is configured to be positioned on a first surface of an imaging subject during use. The marker also includes a second portion of radiolucent material comprising a second radiopaque pattern visually distinguishable from the first radiopaque pattern. The second portion is configured to be positioned on a second generally opposite surface of the subject during use. When radiant energy is emitted through the first portion, the subject, and the second portion to produce a radiograph, the first radiopaque pattern and the second radiopaque pattern produce corresponding first and second shadows on the radiograph. The first shadow and the second shadow are visually distinguishable from one another based on the first radiopaque pattern being visually distinguishable from the second radiopaque pattern, and are usable for identifying characteristics of the radiograph.

US2018/098820A1 describes medical devices and methods that provide for improved accuracy when positioning of a synthetic structure, such as a MEMS device or a stent, within a biological feature of a patient, such as a blood vessel. A blood vessel sizing device is configured for placement on the skin of a patient near a feature of interest (e.g. a blood vessel to be imaged). The device may include one or more radiopaque elements, including a target element, and one or more positioning markers having known sizes. A clinician may use the radiopaque elements to identify a portion of a blood vessel suitable for positioning of the synthetic structure.

US2019/008412A1 describes a system to detect a medical device within a biological subject, the system comprising: an imaging system operable to generate image data derived from the biological subject and image data derived from the medical device; and a processor operable to detect the medical device and evaluate the image data derived from the medical device to identify the nature of the medical device. Further described is a system to match a signature from a detected medical device within a biological subject with signature information held in a databank for a plurality of medical devices, the databank also holding specification information corresponding to the plurality of medical devices, the system being operable to match a signature relating to a detected medical device with signature information held in the databank and to provide some or all of the specification information for the matched signature to identify the nature of the medical device.

In the hospital, workflow for imaging procedures the exact placement of a patient and the adaptation of the scanning position and sequence takes quite some time and a lot of interaction with the clinical staff is required. Manual placement and positioning of the patient is standard, but new supporting tools like optical cameras are becoming available for the general patient position alignment. However, it is not yet possible to mark the exact position at the body of a patient to be scanned and the scanner then detects that region of interest automatically.

There is a need to address these issues.

### SUMMARY OF THE INVENTION

The invention is as defined in the appended claims. Embodiments, examples or aspects in the following disclosure which do not fall under the scope of the claims are presented for illustration purposes only and do not form part of the invention.

It would be advantageous to have improved means of acquiring image data of a patient. The object of the present invention is solved with the subject matter of the independent claims, wherein further embodiments are incorporated in the dependent claims. It should be noted that the following described aspects and examples of the invention apply also to the patient marker, imaging system, and method of imaging a patient with an imaging system, as well as for the computer program element and computer readable medium.

According to a first aspect, there is provided a patient marker. The marker is configured to be placed on or inside a patient. The marker comprises a specific structure. When the marker is placed on or inside the patient, and the patient is positioned at least partially within an image acquisition unit of an imaging system, the marker is configured such that an image acquired by the imaging system comprises image data of the specific structure of the marker. The marker is configured such that image data of the specific structure of the marker comprises information useable to identify the marker. The marker is configured such that image data of the specific structure of the marker comprises information useable to define at least one parameter relating to an examination of the patient.

In other words, a new marker is provided that has a structure that is visible by the imaging system in order to identify the marker and hence the patient and define information such as a region of interest of the patient and how the scanner should be set up etc. In this way, for example a scout scan can be used from which image data of the marker is acquired, and from which a region of interest of the patient can be defined and other information relating to the patient and how the imaging system should operate can be defined. The imaging system can then acquire the required imagery of the region of interest in a scan sequence for example, where the imaging system is operating as required.

Thus, a marker can be placed by a doctor some time in advance and the image acquisition can be conducted in an autonomous way.

In this way, image acquisition set-up and alignment is automatically enabled, as the imaging system on the basis of image data of the marker can configure itself to acquire image data of the required region of interest of the patient, significantly improving the imaging workflow and reliability.

In an example, the marker is configured to be compatible with an image acquisition unit of the imaging system that is one of: an X-ray unit, a CT X-ray unit, an MRI unit.

Thus, a marker can be placed on or in a patient. That patient can be imaged by one imaging protocol, for example using a CT system, and the image data of the marker can be used to enable a particular region of interest of the patient to be imaged and/or enables the imaging system to be set-up for imaging in the required manner. If a doctor then decides that a different imaging protocol, for example MRI, is required the patient can then immediately be taken to an MRI scanner and again image data of the marker can be used to automatically enable the MRI scanner to image the region of interest and be correctly set-up for imaging as required.

In an example, the specific structure of the marker comprises a plurality of regions configured to provide at least two different signal levels in the image data of the specific structure of the marker.

In this way, the marker has a structure that when imaged by an MRI system and/or an X-ray system has different regions that provide different signals levels in the imaging data, and that structural information can be used to define at least one parameter relating to the examination of the patient.

Thus, the same marker can be used in both MRI and x-ray systems such as CT, there can be different absolute signals levels in one imaging system with respect to the other, but what is provided is distinguishable signal levels in each modality enabling data of the marker to be read out.

In an example, the plurality of regions comprises regions of at least two X-ray absorption levels for the image acquisition unit.

In this manner, an x-ray system for example when imaging the marker is provided with image data that has different signal levels, that could be achieved for example by the marker being fabricated from materials having different X-ray attenuation levels and/or with parts of the marker being of different thicknesses and where that marker could be made from a uniform material.

In an example, the plurality of regions comprises regions of a plurality of thicknesses.

In an example, the plurality of regions comprises regions having a ferromagnetic particle.

In this manner, an MRI system for example when imaging the marker is provided with image data that has different signal levels, and ferromagnetic particles can also be sized in order to be visible in x-ray image data.

In an example, the plurality of regions comprises regions having ferromagnetic particles of at least two sizes.

In an example, the specific structure of the marker comprises a pixelated structure.

In an example, the marker in response to an image acquisition by the imaging system is configured to undergo a change, such that image data of the marker in a subsequent image acquisition is different to image data of the marker in a prior image acquisition.

In this manner, it can be determined if the patient has received a scan, and indeed a mechanism is provided to determine time series information relating to where in a series of scans the patient is. Thus, the patient may be anticipated to require 5 sets of scans over 5 days, and after each scan the marker changes in a known manner, and as such it can be determined for example when a fourth set of scans are being undertaken that the patient has already had three prior sets of scans. In this way, automatic time series control is enabled and dosage control is enabled, where for example a scout scan of a sixth set of scans could automatically indicate to the operator that the patient was expected to receive five sets of scans and that they have already received those five sets of scans.

According to a second aspect, there is provided an imaging system, comprising:
- an image acquisition unit; and
- a processing unit.

The image acquisition unit is configured to acquire image data of a patient on whom or within whom is placed a patient marker according to the first aspect. The image data of the patient comprises image data of the specific structure of the marker. The processing unit is configured to identify the marker, the identification comprising utilization of the image data of the specific structure of the marker. The processing unit is configured to define at least one parameter relating to an examination of the patient comprising utilization of the image data of the specific structure of the marker.

In an example, the processing unit is configured to set a scanning protocol and/or parameter setting of the imaging acquisition unit comprising utilization of the image data of the specific structure of the marker.

In other words, the at least one parameter relating to an examination of the patient can be a region of interest to be scanned, and/or how the imaging system is to carry out an imaging examination and how the imaging system will operate during that imaging examination.

In an example, the processing unit is configured to control the image acquisition unit to acquire image data of a region of interest of the patient.

In an example, the processing unit is configured to remove or reduce image data of the marker from the image data of the region of interest of the patient.

In an example, the processing unit is configured to utilize prior information relating to the specific structure of the marker to remove or reduce the image data of the marker from the image data of the region of interest.

According to a third aspect, there is provided a method of imaging a patient with an imaging system, comprising:
- positioning a patient at least partially within an image acquisition unit of the imaging system, wherein a patient marker according to the first aspect is placed on or within the patient;
- acquiring image data of the patient, wherein the image data of the patient comprises image data of the specific structure of the marker;
- identifying the patient by a processing unit, the identification comprising utilization of the image data of the specific structure of the marker; and
- defining at least one parameter relating to an examination of the patient, the defining comprising utilization of the image data of the specific structure of the marker.

According to another aspect, there is provided a computer program element controlling a system as previously described which, if the computer program element is executed by a processing unit, is adapted to perform the method steps as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a patient marker;
Fig. 2 shows a schematic set up of an example of an imaging system;
Fig. 3 shows a method of imaging a patient with an imaging system;
Fig. 4 shows an example of a patient marker;
Fig. 5 shows a representation of an exemplar specific structure of a patient marker; and

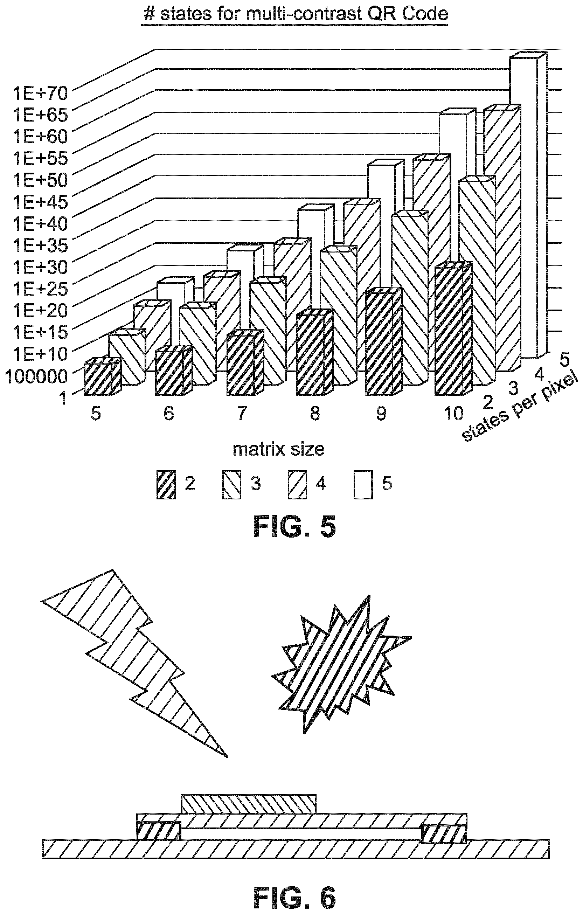

Fig. 5 shows a schematic example of a patient marker.

### DETAILED DESCRIPTION OF EMBODIMENTS

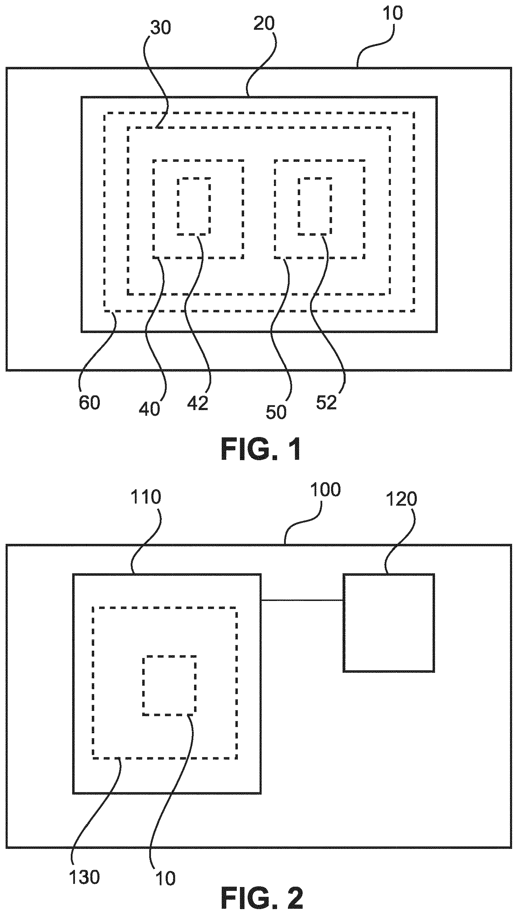

Fig. 1 shows an example of a patient marker 10. Essential features are drawn in solid lines and optional features are drawn in dashed lines. The marker is configured to be placed on or inside a patient. The marker comprises a specific structure 20. When the marker is placed on or inside the patient, and the patient is positioned at least partially within an image acquisition unit of an imaging system, the marker is configured such that an image acquired by the imaging system comprises image data of the specific structure of the marker. The marker is configured also such that image data of the specific structure of the marker comprises information useable to identify the marker. The marker is configured also such that image data of the specific structure of the marker comprises information useable to define at least one parameter relating to an examination of the patient.

In an example, the image data of the specific structure of the marker comprises information useable to set a scanning protocol and/or parameter setting of the imaging acquisition unit.

In an example, the marker is configured to be placed around a standard part of a patient, for example a patient's wrist.

In an example, the at least one parameter relating to the examination of a patient comprises a definition of a region of interest of the patient.

In an example, the at least one parameter relating to the examination of a patient comprises information about the patient.

In an example, the at least one parameter relating to the examination of a patient comprises information about the scanning protocol of the imaging system.

In an example, the at least one parameter relating to the examination of a patient comprises information about the scanning protocol of an X-ray imaging system.

In an example, the at least one parameter relating to the examination of a patient comprises information about the scanning protocol of a CT X-ray imaging system.

In an example, the at least one parameter relating to the examination of a patient comprises information about the scanning protocol of an attenuation X-ray imaging system.

In an example, the at least one parameter relating to the examination of a patient comprises information about the scanning protocol of an MRI imaging system.

In an example, the at least one parameter relating to the examination of a patient comprises information about a follow up imaging examination to be carried out by a different imaging system. Thus, image data can be acquired by an x-ray system such a CT system and that image data of the marker can be used to set up the CT system to carry out a detailed examination for a known patient, and also to indicate that that patient is then required to have an MRI examination, and vice versa.

In an example, the information useable to identify the marker can be used to identify a patient with whom the marker is being, or is going to be, used.

In an example, the examination of the patient is an image exam that is about to be performed by the imaging system that acquired the initial image data of the marker. Thus, for example a patient can be imaged by an MRI system operating in a "Scout Scan" mode, and where image data of the marker from that Scout Scan can be used to correctly set up the MRI system and determine what part of the patient' body to be imaged and how. Or, a patient can be imaged by an X-ray system operating in a "Scout Scan" mode, and where image data of the marker from that Scout Scan can be used to correctly set up the X-ray system and determine what part of the patient' body to be imaged and how. Indeed, the image data in the marker can be used to set up a different imaging system. Thus, a patient can be imaged in an X-ray system, with a first imaging exam undertaken on the basis of image data from the marker, then the patient can be transferred to a different imaging system, for example an MRI system and either the information retrieved of the marker in the X-ray system can be used to set up the MRI system and determine what part of the patient's body to image and how, or an initial scan can be used to obtain the necessary information from MRI image data of the marker.

According to an example, the marker is configured to be compatible with an image acquisition unit of the imaging system that is one of: an X-ray unit, a CT X-ray unit, an MRI unit.

In an example, the patient marker is configured to be compatible with both an X-ray image acquisition unit and an MRI image acquisition unit.

In an example, a marker is configured to be optimised for an MRI system.

In an example, a marker is configured to be optimised for an X-ray system.

According to an example, the specific structure of the marker comprises a plurality of regions 30 configured to provide at least two different signal levels in the image data of the specific structure of the marker.

According to an example, the plurality of regions comprises regions of at least two X-ray absorption levels 40 for the image acquisition unit.

In an example, the plurality of regions comprises regions of three or more absorption levels.

According to an example, the plurality of regions comprises regions of a plurality of thicknesses 42.

According to an example, the plurality of regions comprises regions having a ferromagnetic particle 50.

In an example, a particle is spherically sized, thus providing for an angular independent signal level.

In an example, the ferromagnetic particle is a ferromagnetic platelet.

According to an example, the plurality of regions comprises regions having ferromagnetic particles of at least two sizes 52.

In an example, the plurality of regions comprises regions having ferromagnetic platelets of three or more sizes.

According to an example, the specific structure of the marker comprises a pixelated structure 60.

In an example, the specific structure comprises a QR like structure.

According to an example, the marker in response to an image acquisition by the imaging system is configured to undergo a change, such that image data of the marker in a subsequent image acquisition is different to image data of the marker in a prior image acquisition.

In an example, the marker is 3D printable.

Thus, for example new multi material 3D printing technologies allow voxel based printing of different materials that enables 3D information to be provided in the marker.

In an example, the marker is compatible with an MRI image acquisition unit and is configured to be coded and/or programmed by an RF pulse from the MRI image acquisition unit.

Fig. 2 shows an example of an imaging system 100. The imaging system comprises an image acquisition unit 110, and a processing unit 120. The image acquisition unit is configured to acquire image data of a patient 130. On the patient or within the patient is placed a patient marker 10 as described above with respect to Fig. 1. The image data of the patient, when acquired, comprises image data of the specific structure of the marker. The processing unit is configured to identify the marker. The identification comprises utilization of the image data of the specific structure of the marker. The processing unit is configured also to define at least one parameter relating to an examination of the patient. The definition comprises utilization of the image data of the specific structure of the marker.

In an example, the image acquisition unit is one of: an X-ray unit, a CT X-ray unit, an MRI unit.

In an example, the marker is placed adjacent to a region of interest of the patient.

In an example, the marker is placed covering at least a part of the region or interest.

According to an example, the processing unit is configured to set a scanning protocol and/or parameter setting of the imaging acquisition unit comprising utilization of the image data of the specific structure of the marker.

According to an example, the processing unit is configured to control the image acquisition unit to acquire image data of a region of interest of the patient.

According to an example, the processing unit is configured to remove or reduce image data of the marker from the image data of the region of interest of the patient.

According to an example, the processing unit is configured to utilize prior information relating to the specific structure of the marker to remove or reduce the image data of the marker from the image data of the region of interest.

In an example, the imaging system comprises a visible camera and wherein the marker is configured to be placed on the patient, and wherein the image data of the specific structure of the marker is in a visible image format.

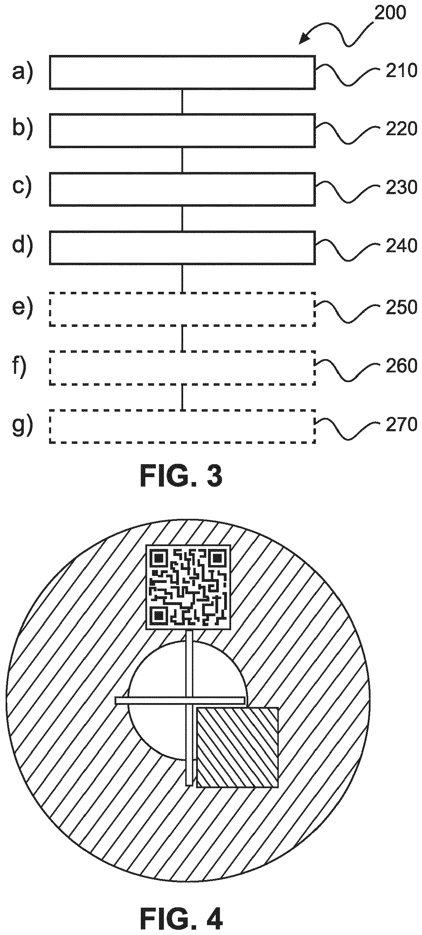

Fig. 3 shows a method 200 of imaging a patient with an imaging system in its basic steps, where essential steps are shown in bold lines and optional steps are shown in dashed lines. The method comprises:
- in a positioning step 210, also referred to as step a), positioning a patient at least partially within an image acquisition unit of the imaging system, wherein a patient marker as described with respect to Fig. 1 is placed on or within the patient;
- in an acquiring step 220, also referred to as step b), acquiring image data of the patient, wherein the image data of the patient comprises image data of the specific structure of the marker;
- in an identifying step 230, also referred to as step c), identifying the patient by a processing unit, the identification comprising utilization of the image data of the specific structure of the marker; and
- in a defining step 240, also referred to as step d), defining at least one parameter relating to an examination of the patient, the defining comprising utilization of the image data of the specific structure of the marker.

In an example, the image acquisition unit is one of: an X-ray unit, a CT X-ray unit, an MRI unit.

In an example, the method comprises step e) setting 250 a scanning protocol and/or parameter setting of the imaging acquisition unit by the processing unit comprising utilization of the image data of the specific structure of the marker.

In an example, the method comprises step f) controlling 260 by the processing unit the image acquisition unit to acquire image data of a region of interest of the patient comprising utilization of the image data of the specific structure of the marker .

In an example, the method comprises step g) removing or reducing 270 image data of the marker from the image data of a region of interest of the patient by the processing unit.

In an example, step g) comprises utilization of prior information relating to the specific structure of the marker.

The patient marker, imaging system and method of imaging a patient with an imaging system are now described in more detail, where reference is made to Figs. 4-6.

As discussed above, a new marker is provided that can be visible in X-ray, CT and MRI imaging modalities and/or in an image acquired by a visible camera associated with such diagnostic imaging modalities. The marker can have a QR-code like information in it that includes information that identifies the marker, and has patient specific information as well as imaging procedure relevant information. The information is visible in the MRI image as well as in the X-ray/CT image and can be read out and used by one or more of these imaging systems. However, a marker can be made to be better suited for one of these imaging modalities, but still function in a different imaging modality. Additional location indicating elements define the region of interest at the patient when the markers are placed on the patient's body and then imaged. The placement can be done by a doctor, some time in advance, and the image acquisition can be done in an autonomous way as all relevant information about position, scan protocol and patient information is stored in the patient specific marker. New multi material 3D printing technologies allow voxel based printing of different materials that enables a 3D information unit in the marker.

Thus, the patient markers has sections with material properties that are placed such that when viewed in an imaging mode of a scanner, they appear to be coded with information relevant for the scan, such as the patient information, the area of the patient to be scanned, how the scanner should be set up. As discussed above, in some embodiments, the patient marker can be coded in a similar manner to that for a QR code.

The patient marker, an example of which is shown in Fig. 4, is visible in image data acquired by x-ray, CT and MRI systems - either by the diagnostic imaging modality itself and/or in image data acquired by a visible camera used as part of these diagnostic imaging systems. The information in the image data of the marker includes the exact position visibility of a reference point, as well as the patient identification and procedure information (such as where to and how to conduct imaging) that is coded in an imaging system readable way. As discussed, this can be done either by additional optical cameras in a QR-code like way, or in a voxel based code that has for example different x-ray absorption properties per voxel and/or different MRI visibilities, but this can still be viewed as a QR like mode of information content.

The QR like code can be printed in an optical visible way, and also can be 3D-printed, with materials that can have similar optical properties, but have different x-ray absorption and/or different MRI contrast due to the different material properties per 3D-printed voxel. (MRI spectrum close to water, CT e.g. plastic filled with metal powder or different types of plastic).

3D printing of different materials is possible for example with Polyjet printers from Stratasys (e.g. J750), but also with other 3D printers that can handle multiple materials or that can print different material densities. Either selecting material A or material B per voxel allows for digital coding and/or the mix of materials even allows for linear coding. The selection of the materials can be done to obtain a maximum contrast for x-ray, CT / MR imaging. A desk-top 3D printer can print the marker, or at least the patient/position specific part of the marker, just in time, in other words as the patient arrives for imaging. The marker can also be designed in order that it provides a link to a planned patient sequence of scans. A tracking system can be utilized to keep track of a list of markers to print from the hospital system as per the required priority.

Thus, the marker can include the information related to focus of the scan along with patient identification and position. The information stored in the marker can therefore speed up the process to improve the workflow and reliability of the scan.

The marker can be used to provide unique identification of the region of interest for example, having a code for "upper left", "upper right", "lower left" and "lower right" to define the area that has to be scanned. More detailed information, such as a specific part of an organ to be imaged can be encoded. Different unique geometries in 2D / 3D can be provided, for example as shown in fig. 4 where a solid square is positioned at one quadrant of the area centred on a cross, in relation to an absolute reference position - the centre of the cross for example. Also, as shown in Fig, 4 the marker image data can be seen by the scanner as a QR square, that provides information as to where to scan, that can be in relation to the reference centre cross position (or not) and contains information such as the patient's name, and how the scanner should be set up for scanning. Again, this can be done with unique materials at special positions. The marker could be applied to the patient's body like a temporal tattoo or like a plaster.

Thus, a specific example of a workflow for autonomous imaging is the following:
Step 1: the doctor prepares the imaging together with the patient by sticking the marker at the patient's body at exactly the positions defining the region of interest. This process can be further automated by a 3D printer that prints and/or marks a stamp on the patient. The other option is to have printing at same standard location (for example on wrist) and the 3D object or stamp is provided by a machine in the form of an automated cuff or a printer nozzle can spray the readable marker.
Step 2: hours or days later (that can be important for optimization of efficiency and/or flexibility for patient and usage of an imaging system), the patient goes to the imaging system that automatically positions the patient at the region of interest and acquires the images. Patient data and scan protocol information is available in the scanner, but also in the marker. This helps to increase the safety that all parameters are correct and the right person is scanned.

### Embodiment: multi-contrast OR code

The marker as discussed above can encode several types of information. The required number of marker states can be estimated as the product of the following quantities, resulting in about 10²⁵.
patient identification unique for all humans and over few decades (10x world population):
   10¹¹
up to 10 scans are typically done, with scan protocol and FOV required per scan:
   10
FOV relative to marker position (xmin = -20, xmax = 20, ymin, ..., range= ±1000mm):
   12000
Scan protocol out of a list of MR and CT protocols:
   250
Patient conditions can be encoded separately for safety:
   100

For example: critical clinical states, implants, paediatric/demented/.., overall fitness, breath-hold duration, to be sedated, special attention (by robot),
hash functions / checksums to ensure data integrity at 0.1% ambiguity rate:
   1000
margin for future data extension:
   100

Multiplying the above numbers gives the required number of marker states of about 10²⁵

In a CT imaging modality the information in the marker can be read with CT scout scan, which is performed at the begin of the examination. Otherwise, additional dose would be applied to the patient.

In a MRI imaging modality, standard scout scans cannot directly read out the marker due to an out-of-slice problem (see details in embodiment "MR Near-Projection Imaging" below). However, additional scan time for a marker scan should be kept to a minimum, which in turn means that the spatial resolution for this scan should be kept low (for example 0.5cm to 1cm resolution). Figure 5 shows that standard QR codes would require at least a 10x10 pixel wide matrix to encode all required states, which at a scan resolution of 1cm would mean at least a 10cmx10cm large adhesive patch. To limit the size of the patch more than two different contrast states can be used for each pixel. The number of possible different QR codes exploiting this feature is plotted in Figure 5.

Thus, referring to Fig. 5 this shows the information content of a marker in a QR code like format. Here, Fig. 5 shows the number of possible different QR codes as a function of square matrix size and number of contrast states per pixel. Note the logarithmic scale. The required number of 10²⁶ would require a 10x10 pixel matrix for conventional 2-state-matrices and only 6x6 pixels for five different pixel contrasts. Thus, in the QR like code marker developed here there is a slight difference in that unlike a normal QR code used for example on groceries that has only two information states (black or white), here the QR code format in the imaging modalities discussed here can have numerous information states. Thus, in X-ray (and visible in MRI too) a QR code can have 2, 3, 4, or 5 or more different thicknesses, providing for a QR code marker having information in the third dimension of "thickness" or absorption - as different materials with different absorptions can be used. Also, in a MRI (visible in X-ray too ) imaging modalities, the QR code marker can have a grid of pixels as shown in Fig. 5, from 5x5 to 10x10 for example, Then at the centre of each pixel a ferromagnetic platelet can be positioned. Thus, there can be two states - ferromagnetic platelet of no platelet. However, the platelets can have various sizes in the QR grid, providing for different information states, that have different signals in MRI and can be seen as different sizes in X-ray. Thus, as shown in Fig. 5 when there is an information content of two, a standard QR code marker would require at least a 10x10 pixel wide matrix to encode all required states, which at a scan resolution of 1cm would mean at least a 10cm×10cm large adhesive marker or patch. But, as discussed above the marker can be smaller, and require higher resolution imaging. By having more than two possible contrast states per pixel, this shows that a 6x6 pixel QR code marker with 5 contrast states has the required information content, as detailed above, enabling a marker size to be reduced by a factor of three.

### Embodiment: isolated small ferromagnetic platelets

A CT can identify relatively small high density objects in scout scans because of its good spatial and contrast resolution. In MRI imaging the signal void artefacts of ferromagnetic particles can be made much larger than the particle itself, depending on the material and scan protocol. Therefore, in an embodiment MR/CT markers are provided that do not represent a closed surface covering the full area of 6cmx6cm to 10cm×10cm, but rather a number of separate very small ferromagnetic metal platelets arranged in a matrix of that size. It is possible to make such platelets with a diameter of slightly less than 1mm and a height of about 300µm and still with a 1cm large MR signal void and visible in CT, and as disused above the platelets can have different sizes to provide a third dimension of information content in both CT, X-ray and MRI. However, this approach vastly reduces the perceived size of the marker because the actually covered skin area becomes very small. It is further proposed to apply all platelets in one go using a helper patch that is applied to the skin and immediately removed, leaving the arranged platelets separately glued to the skin.

Also, the 10cm×10cm or 6cmx6cm sized patch can again be sized to refer to a scout scan "first step" methodology, but this is not essential and these imaging modalities can operate in a normal manner to acquire the marker at high resolution, enabling the marker to be much smaller in size.

Alternatively, fine dust metal particle in the form of desired QR arrangements can be printed via a printer, that can be scouted using a UV or near UV camera thus avoiding the x-ray dosage.

### Embodiment: MR (near-)projection imaging

A CT scout scan typically involves a single anterior-posterior and/or left-right projection image. These projections can then include the marker. As stated above, it can be desirable for MR scout scans typically to comprise stacks of a few slices, with gaps of the order of 2-4 slice thicknesses in-between. Such scans, in general, may not then cover the full marker, but it may at least, in part, be located within the gaps. Exploiting the fact that in an embodiment when the marker is known to be present at the skin (and not within the body), the following approach can be utilized: the initial standard scout scan is used to derive a very simple geometric model of the exterior hull of the patient. Subsequently, the system automatically plans an additional marker scan to cover the skin of the patient, with one or more thick slices. Full MR projections (i.e., no slice selection at all) may be used for this marker scan as well, but thick slices will enable a better contrast resolution. In this manner, an additional marker scan may be tolerable as long as it is very short.

### Fluorescent Markers / Changing markers

The marker can be composed in such a way that it can change colour, texture etc. after the imaging has been performed. Interaction of different materials (e.g. sterilization indicators) with ionizing radiation, strong magnetic fields, RF waves etc. is used as a control that the imaging has been performed and that the field-of-view was set correctly, i.e. a change in colour/texture (or other change) only applies when the x-ray beam covers the marked area. In MRI, this can also include capsules with switchable contrast agents [see for example US9786420] sensitive to the RF frequency of the MRI, temperature etc. This allows for the detection of successful imaging, from the acquired images. The marker can be equipped with a fluorescent identification (QR code, etc). This identification can also be printed using a 3D printer. A fluorescent optical signal can also be visible under tissue. An in-bore light source induces visibility of the fluorescent id tags, and the fluorescent marker is also visible in the x-ray regime. As shown in Fig. 6, which shows a marker with a fluorescent identifier, the marker can be attached to a thin flexible sticker attached to the skin.

A smart dedicated printer device can store, provide and program the dedicated patient specific identifier. Corresponding QR-codes/RF id or fluorescent ids are printed or attached on the MR/CT marker. Such a device can be located in the operator room, but can also be located close to the diagnostic scanner. A programmable tag device can also be integrated at the front of the MRI/CT scanner. Such a device can also be portable or built as a handheld device. Such a handheld device can directly guide the user to the right position for fixing the marker. A SW interface and program can be used to control the data flow between patient data base of the hospital, diagnostic scanner and marker print device.

Typically, markers with contrast liquid can be somewhat bulky and may be in conflict with therapy and medical processes. Therefore, the tag/marker with the contrast agent can be affixed to skin, garment or coil by stitching, adhesives, mechanical fasteners, or other means to a flat/thin flexible marker base, whicth can remain affixed to a skin surface for an extended period of time. An MR/CT marker can also be implanted/disposed in a body cavity/surgical wound for an extended period of time.

A marker can include an RF coil, and the marker is mechanical fixed to the RF coil using mechanical fasteners. Thus, the marker can act as an RF-id marker as well. Thus, the marker can be coded/programmed by a RF pulse sequence of the MR system. The RF pulse sequence can program an electronic circuit linked to the RF coil on the marker, which allows to set the marker in a special state (on/off). This enables a record to be made that the patient was exposed to an RF field or was in the magnet. Thus, a marker can change its contrast behavior on demand.

### Embodiment: AI-based readout of markers

The readout process for the markers is robust and can be performed in few seconds. Thus, a result of the marker readout is available to define further diagnostic scans in the same examination. The spatial structures of the marker can be quite different from any anatomic structures, for example being a square grid like structure. When the marker itself partially overlays the region of interest, a human observer can then identify what is the marker and what is the anatomical structure in the image. However, as discussed above image processing can be used to reduce or remove the effect of the marker in the image, which can be helped when the standard form of the marker is known - for example when the pixel to pixel spacings are known. Thus, the subtraction of the "marker out of the image" as one example, in CT imaging modality a forward projection of the marker object can be done if the position, 3D geometry and material composition of the marker is known (from a database or via info from the identified marker ID). The forward projected "simulated marker image" can then be subtracted from the acquired image and the marker would not be visible. Also, methods of iterative reconstruction can be used to compensate, from knowledge of which defined and known object should be "not visible". However, robust and fast read out of the marker image data can in certain situations not be simple for standard image processing means, because contrast and projected shape of the marker may differ considerably with marker location on the body and per patient. Therefore, a trained convolutional neural network (or similar network) is used with correctly labelled training images obtained from clinical scans, with further training images be generated from these by image transformations. The label is defined as a vector comprising all pixels of the marker matrix with a respective discrete value range (see embodiment for multi-contrast QR code). The inference of the trained network with respective initial CT and MR images as input is used to read out the marker content in clinical practice. Inference engines such as open VINO / TensorRT can be utilized to have faster inference with the trained model. This process involves the optimizations carried out at network architecture level, batch normalizations and low level computations using math kernel libraries. This allows the inference to run on multiple platforms such as directly in MR/CT boxes, CPUs, FPGAs or even in USB stick with more throughput / frames per second. This contributes to improvement of workflow with the markers in autonomous scan, because the marker can be accurately read out wherever it is placed on the patient.

Machine-learning techniques are also used, based on historical data to classify the type of markers and to optimize what to print on the markers. This helps to identify focus of the scan, position for a particular scan and protocol in autonomous way to complement the doctor to define the region and suggest the type of marker for the scan. This can be followed by compression techniques to minimize the information to print on the marker, as the size of the marker and faster processing can be important factors.

### Embodiments: Time Series Scan

In many clinical conditions, a patient has to go for repeat scans for the same anatomical region to check the progression of clinical conditions. For example, for a patient with neuro-critical conditions where a brain swelling may shift the midline, with scanning having to be performed repeatedly to check the swelling conditions. The above marker, for example the wearable marker can also be encoded with time specific information so that repeat scans can be performed with the same settings. This will enable a more predictable and repeatable scans. Furthermore, as discussed above the progression of the patient through a set of scans can be followed, where the marker changes after each scan (colour/texture etc), where it then be established how many scans the patient has had. The degree/amount of contrast/florescent quantity in the marker can be decided upon based on the expected number of scans required at a specific location, so that a time decay based or scan decay based QR code can be created appropriately.

### Summary

Thus, in summary a specific detailed marker is provided that:
is visible in the image
has a code for patient specific identification
has a position feature for definition of the region of interest
has additional information about scanning protocols and parameter settings
has all information readable by the imaging system in an automatic way
has all information readable by the imaging method of the imaging system in a way that the information is also available in the acquired image data (e.g. coded in x-ray absorption or MRI contrast)

Can provide time series information, for e.g. repeat scans
However, markers can be provided, as described above that do not need to provide all of this functionality.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A patient marker (10):
- wherein, the marker is configured to be placed on or inside a patient;
- wherein, the marker comprises a specific structure (20);
- wherein, when the marker is placed on or inside the patient, and the patient is positioned at least partially within an image acquisition unit of an imaging system, wherein the marker is compatible with both the image acquisition unit when it is an X-ray unit and with the image acquisition unit when it is an MRI unit, and wherein the marker is configured such that an image acquired by the imaging system comprises image data of the specific structure of the marker;
- wherein, the marker is configured such that image data of the specific structure of the marker comprises information useable to identify the marker when the image acquisition unit is the X-ray unit and the marker is configured such that image data of the specific structure of the marker comprises information useable to identify the marker when the image acquisition unit is the MRI unit; and
- wherein, the marker is configured such that image data of the specific structure of the marker comprises information useable to define at least one parameter relating to an examination of the patient comprising information useable to set a scanning protocol and/or parameter setting of the image acquisition unit when the image acquisition unit is the X-ray unit and the marker is configured such that image data of the specific structure of the marker comprises information useable to define at least one parameter relating to an examination of the patient comprising information useable to set a scanning protocol and/or parameter setting of the image acquisition unit when the image acquisition unit is the MRI unit.

2. Patient marker according to claim 1, wherein the specific structure of the marker comprises a plurality of regions (30) configured to provide at least two different signal levels in the image data of the specific structure of the marker.

3. Patient marker according to claim 2, wherein the plurality of regions comprises regions of at least two X-ray absorption levels (40) for the image acquisition unit.

4. Patient marker according to any of claims 2-3, wherein the plurality of regions comprises regions of a plurality of thicknesses (42).

5. Patient marker according to claim 2, wherein the plurality of regions comprises regions having a ferromagnetic particle (50).

6. Patient marker according to claim 5, wherein the plurality of regions comprises regions having ferromagnetic particles of at least two sizes (52).

7. Patient marker according to any of claims 1-6, wherein the specific structure of the marker comprises a pixelated structure (60).

8. Patient marker according to any of claims 1-7, wherein the marker in response to an image acquisition by the imaging system is configured to undergo a change, such that image data of the marker in a subsequent image acquisition is different to image data of the marker in a prior image acquisition.

9. An imaging system (100), comprising:
- a plurality of image acquisition units (110); and
- a processing unit (120);
wherein, the plurality of image acquisition units comprises an X-ray unit and an MRI unit;
wherein, each image acquisition unit is configured to acquire image data of a patient (130) on whom or within whom is placed a patient marker (10) according to any of claims 1-8;
wherein, the image data of the patient comprises image data of the specific structure of the marker;
wherein, the processing unit is configured to identify the marker, the identification comprising utilization of the image data of the specific structure of the marker when the image acquisition unit is the X-ray unit, and the processing unit is configured to identify the marker, the identification comprising utilization of the image data of the specific structure of the marker when the image acquisiiton unit is the MRI unit; and
wherein, the processing unit is configured to define at least one parameter relating to an examination of the patient comprising to set a scanning protocol and/or parameter setting of the image acquisition unit comprising utilization of the image data of the specific structure of the marker when the image acquisiiton unit is the X-ray unit, and the processing unit is configured to define at least one parameter relating to an examination of the patient comprising to set a scanning protocol and/or parameter setting of the image acquisition unit comprising utilization of the image data of the specific structure of the marker when the image acquisiiton unit is the MRI unit.

10. Imaging system according to claim 9, wherein the processing unit is configured to control the image acquisition unit to acquire image data of a region of interest of the patient.

11. Imaging system according to claim 10, wherein the processing unit is configured to remove or reduce image data of the marker from the image data of the region of interest of the patient.

12. Imaging system according to claim 11, wherein the processing unit is configured to utilize prior information relating to the specific structure of the marker to remove or reduce the image data of the marker from the image data of the region of interest.

13. A method (200) of imaging a patient with an imaging system, comprising:
- positioning (210) a patient at least partially within a plurality of image acquisition units of the imaging system, wherein a patient marker according to any of claims 1-9 is placed on or within the patient, and wherein the plurality of image acquisition units comprises an X-ray unit or an MRI unit;
- acquiring (220) image data of the patient, wherein the image data of the patient comprises image data of the specific structure of the marker;
- identifying (230) the patient by a processing unit, the identification comprising utilization of the image data of the specific structure of the marker when the image acquisition unit is the X-ray unit and identifying the patient by the processing unit, the identification comprising utilization of the image data of the specific structure of the marker when the image acquisition unit is the MRI unit; and
- defining (240) at least one parameter relating to an examination of the patient comprising setting (250) a scanning protocol and/or parameter setting of the imaging acquisition unit, the defining comprising utilization of the image data of the specific structure of the marker when the image acquisition unit is the X-ray unit and defining at least one parameter relating to an examination of the patient comprising setting a scanning protocol and/or parameter setting of the imaging acquisition unit, the defining comprising utilization of the image data of the specific structure of the marker when the image acquisition unit is the MRI unit.

## Patentansprüche

1. Patientenmarker (10):
- wobei der Marker konfiguriert ist, um an oder in einem Patienten platziert zu werden;
- wobei der Marker eine spezifische Struktur (20) umfasst;
- wobei, wenn der Marker an oder in dem Patienten platziert ist und der Patient zumindest teilweise innerhalb einer Bilderfassungseinheit eines Bildgebungssystems positioniert ist, wobei der Marker sowohl mit der Bilderfassungseinheit, wenn sie eine Röntgeneinheit ist, als auch mit der Bilderfassungseinheit, wenn sie eine MRI-Einheit ist, kompatibel ist, und wobei der Marker konfiguriert ist, sodass ein Bild, das durch das Bildgebungssystem erfasst wird, Bilddaten der spezifischen Struktur des Markers umfasst;
- wobei der Marker konfiguriert ist, sodass Bilddaten der spezifischen Struktur des Markers Informationen umfassen, die zum Identifizieren des Markers verwendet werden können, wenn die Bilderfassungseinheit die Röntgeneinheit ist, und der Marker konfiguriert ist, sodass Bilddaten der spezifischen Struktur des Markers Informationen umfassen, die zum Identifizieren des Markers verwendet werden können, wenn die Bilderfassungseinheit die MRI-Einheit ist; und
- wobei der Marker konfiguriert ist, sodass Bilddaten der spezifischen Struktur des Markers Informationen umfassen, die zum Definieren mindestens eines Parameters in Bezug auf eine Untersuchung des Patienten verwendet werden können, umfassend Informationen, die zum Einstellen eines Scan-Protokolls und/oder einer Parametereinstellung der Bilderfassungseinheit verwendet werden können, wenn die Bilderfassungseinheit die Röntgeneinheit ist, und der Marker konfiguriert ist, sodass Bilddaten der spezifischen Struktur des Markers Informationen umfassen, die zum Definieren mindestens eines Parameters in Bezug auf eine Untersuchung des Patienten verwendet werden können, umfassend Informationen, die zum Einstellen eines Scan-Protokolls und/oder einer Parametereinstellung der Bilderfassungseinheit verwendet werden können, wenn die Bilderfassungseinheit die MRI-Einheit ist.

2. Patientenmarker nach Anspruch 1, wobei die spezifische Struktur des Markers eine Vielzahl von Bereichen (30) umfasst, die konfiguriert sind, um mindestens zwei unterschiedliche Signalpegel in den Bilddaten der spezifischen Struktur des Markers bereitzustellen.

3. Patientenmarker nach Anspruch 2, wobei die Vielzahl von Bereichen Bereiche von mindestens zwei Röntgenabsorptionsniveaus (40) für die Bilderfassungseinheit umfasst.

4. Patientenmarker nach einem der Ansprüche 2 bis 3, wobei die Vielzahl von Bereichen Bereiche mit einer Vielzahl von Stärken (42) umfasst.

5. Patientenmarker nach Anspruch 2, wobei die Vielzahl von Bereichen Bereiche mit einem ferromagnetischen Partikel (50) umfasst.

6. Patientenmarker nach Anspruch 5, wobei die Vielzahl von Bereichen Bereiche mit ferromagnetischen Partikeln von mindestens zwei Größen (52) umfasst.

7. Patientenmarker nach einem der Ansprüche 1-6, wobei die spezifische Struktur des Markers eine gepixelte Struktur (60) umfasst.

8. Patientenmarker nach einem der Ansprüche 1-7, wobei der Marker als Reaktion auf eine Bilderfassung durch das Bildgebungssystem konfiguriert ist, um eine Veränderung zu erfahren, sodass sich die Bilddaten des Markers bei einer nachfolgenden Bilderfassung von den Bilddaten des Markers bei einer vorherigen Bilderfassung unterscheiden.

9. Bildgebungssystem (100), umfassend:
- eine Vielzahl von Bilderfassungseinheiten (110); und
- eine Verarbeitungseinheit (120);
wobei die Vielzahl der Bildaufnahmeeinheiten eine Röntgeneinheit und eine MRI-Einheit umfasst; wobei jede Bilderfassungseinheit konfiguriert ist, um Bilddaten eines Patienten (130) zu erfassen, an oder in dem ein Patientenmarker (10) nach einem der Ansprüche 1-8 platziert ist; wobei die Bilddaten des Patienten Bilddaten der spezifischen Struktur des Markers umfassen; wobei die Verarbeitungseinheit konfiguriert ist, um den Marker zu identifizieren, die Identifizierung umfassend eine Verwendung der Bilddaten der spezifischen Struktur des Markers, wenn die Bilderfassungseinheit die Röntgeneinheit ist, und die Verarbeitungseinheit konfiguriert ist, um den Marker zu identifizieren, die Identifizierung umfassend eine Verwendung der Bilddaten der spezifischen Struktur des Markers, wenn die Bilderfassungseinheit die MRI-Einheit ist; und wobei die Verarbeitungseinheit konfiguriert ist, um mindestens einen Parameter in Bezug auf eine Untersuchung des Patienten zu definieren, umfassend ein Einstellen eines Scan-Protokolls und/oder einer Parametereinstellung der Bilderfassungseinheit, umfassend eine Verwendung der Bilddaten der spezifischen Struktur des Markers, wenn die Bilderfassungseinheit die Röntgeneinheit ist, und die Verarbeitungseinheit konfiguriert ist, um mindestens einen Parameter in Bezug auf eine Untersuchung des Patienten zu definieren, umfassend ein Einstellen eines Scan-Protokolls und/oder einer Parametereinstellung der Bilderfassungseinheit, umfassend eine Verwendung der Bilddaten der spezifischen Struktur des Markers, wenn die Bilderfassungseinheit die MRI-Einheit ist.

10. Bildgebungssystem nach Anspruch 9, wobei die Verarbeitungseinheit konfiguriert ist, um die Bilderfassungseinheit zu steuern, um Bilddaten eines Bereichs von Interesse des Patienten zu erfassen.

11. Bildgebungssystem nach Anspruch 10, wobei die Verarbeitungseinheit konfiguriert ist, um Bilddaten des Markers aus den Bilddaten des Bereichs von Interesse des Patienten zu entfernen oder zu reduzieren.

12. Bildgebungssystem nach Anspruch 11, wobei die Verarbeitungseinheit konfiguriert ist, um vorherige Informationen in Bezug auf die spezifische Struktur des Markers zu verwenden, um die Bilddaten des Markers aus den Bilddaten des Bereichs von Interesse zu entfernen oder zu reduzieren.

13. Verfahren (200) zur Bildgebung eines Patienten mit einem Bildgebungssystem, umfassend:
- Positionieren (210) eines Patienten zumindest teilweise innerhalb einer Vielzahl von Bilderfassungseinheiten des Bildgebungssystems, wobei ein Patientenmarker nach einem der Ansprüche 1 bis 9 an oder innerhalb des Patienten platziert wird und wobei die Vielzahl von Bilderfassungseinheiten eine Röntgeneinheit oder eine MRI-Einheit umfasst;
- Erfassen (220) von Bilddaten des Patienten, wobei die Bilddaten des Patienten Bilddaten der spezifischen Struktur des Markers umfassen;
- Identifizieren (230) des Patienten durch eine Verarbeitungseinheit, die Identifizierung umfassend eine Verwendung der Bilddaten der spezifischen Struktur des Markers, wenn die Bilderfassungseinheit die Röntgeneinheit ist, und Identifizieren des Patienten durch die Verarbeitungseinheit, die Identifizierung umfassend eine Verwendung der Bilddaten der spezifischen Struktur des Markers, wenn die Bilderfassungseinheit die MRI-Einheit ist; und
- Definieren (240) mindestens eines Parameters, der sich auf eine Untersuchung des Patienten bezieht, umfassend ein Einstellen (250) eines Scan-Protokolls und/oder einer Parametereinstellung der Bilderfassungseinheit, wobei das Definieren eine Verwendung der Bilddaten der spezifischen Struktur des Markers umfasst, wenn die Bilderfassungseinheit die Röntgeneinheit ist, und Definieren mindestens eines Parameters, der sich auf eine Untersuchung des Patienten bezieht, umfassend ein Einstellen eines Scan-Protokolls und/oder einer Parametereinstellung der Bilderfassungseinheit, wobei das Definieren eine Verwendung der Bilddaten der spezifischen Struktur des Markers umfasst, wenn die Bilderfassungseinheit die MRI-Einheit ist.

## Revendications

1. Marqueur patient (10):
- dans lequel, le marqueur est configuré pour être placé sur ou à l'intérieur d'un patient;
- dans lequel, le marqueur comprend une structure spécifique (20);
- dans lequel, lorsque le marqueur est placé sur ou à l'intérieur du patient, et que le patient est positionné au moins partiellement à l'intérieur d'une unité d'acquisition d'images d'un système d'imagerie, dans lequel le marqueur est compatible à la fois avec l'unité d'acquisition d'images lorsqu'il s'agit d'un appareil à rayons X et avec l'unité d'acquisition d'images lorsqu'il s'agit d'une unité MRI, et dans lequel le marqueur est configuré de sorte qu'une image acquise par le système d'imagerie comprend des données d'image de la structure spécifique du marqueur;
- dans lequel, le marqueur est configuré de telle sorte que les données d'image de la structure spécifique du marqueur comprennent des informations utilisables pour identifier le marqueur lorsque l'unité d'acquisition d'image est l'unité à rayons X et le marqueur est configuré de sorte que les données d'image de la structure spécifique du marqueur comprennent des informations utilisables pour identifier le marqueur lorsque l'unité d'acquisition d'images est l'unité MRI; et
- dans lequel, le marqueur est configuré de sorte que les données d'image de la structure spécifique du marqueur comprennent des informations utilisables pour définir au moins un paramètre relatif à un examen du patient comprenant des informations utilisables pour définir un protocole de numérisation et/ou un paramétrage de l'image unité d'acquisition lorsque l'unité d'acquisition d'images est l'unité de radiographie et que le marqueur est configuré de sorte que les données d'image de la structure spécifique du marqueur comprennent des informations utilisables pour définir au moins un paramètre relatif à un examen du patient comprenant des informations utilisables pour définir un protocole de balayage et/ou un paramétrage de l'unité d'acquisition d'images lorsque l'unité d'acquisition d'images est l'unité MRI.

2. Marqueur patient selon la revendication 1, dans lequel la structure spécifique du marqueur comprend une pluralité de régions (30) configurées pour fournir au moins deux niveaux de signal différents dans les données d'image de la structure spécifique du marqueur.

3. Marqueur patient selon la revendication 2, dans lequel la pluralité de régions comprend des régions d'au moins deux niveaux d'absorption de rayons X (40) pour l'unité d'acquisition d'images.

4. Marqueur de patient selon l'une quelconque des revendications 2 à 3, dans lequel la pluralité de régions comprend des régions d'une pluralité d'épaisseurs (42).

5. Marqueur patient selon la revendication 2, dans lequel la pluralité de régions comprend des régions ayant une particule ferromagnétique (50).

6. Marqueur patient selon la revendication 5, dans lequel la pluralité de régions comprend des régions ayant des particules ferromagnétiques d'au moins deux tailles (52).

7. Marqueur de patient selon l'une quelconque des revendications 1 à 6, dans lequel la structure spécifique du marqueur comprend une structure pixélisée (60).

8. Marqueur de patient selon l'une quelconque des revendications 1 à 7, dans lequel le marqueur en réponse à une acquisition d'image par le système d'imagerie est configuré pour subir un changement, de sorte que les données d'image du marqueur dans une acquisition d'image ultérieure soient différentes des données d'image du marqueur lors d'une acquisition d'image préalable.

9. Un système d'imagerie (100), comprenant:
- une pluralité d'unités d'acquisition d'images (110); et
- une unité de traitement (120);
dans lequel, la pluralité d'unités d'acquisition d'images comprend une unité à rayons X et une unité MRI; dans lequel, chaque unité d'acquisition d'image est configurée pour acquérir des données d'image d'un patient (130) sur lequel ou à l'intérieur duquel est placé un marqueur de patient (10) selon l'une quelconque des revendications 1 à 8; dans lequel, les données d'image du patient comprennent des données d'image de la structure spécifique du marqueur; dans lequel, l'unité de traitement est configurée pour identifier le marqueur, l'identification comprenant l'utilisation des données d'image de la structure spécifique du marqueur lorsque l'unité d'acquisition d'image est l'unité à rayons X, et l'unité de traitement est configurée pour identifier le marqueur, l'identification comprenant l'utilisation des données d'image de la structure spécifique du marqueur lorsque l'unité d'acquisition d'image est l'unité MRI; et dans lequel, l'unité de traitement est configurée pour définir au moins un paramètre relatif à un examen du patient comprenant le réglage d'un protocole de balayage et/ou le paramétrage de l'unité d'acquisition d'image comprenant l'utilisation des données d'image de la structure spécifique du marqueur lorsque l'unité d'acquisition d'images est l'unité de radiographie, et l'unité de traitement est configurée pour définir au moins un paramètre relatif à un examen du patient comprenant le réglage d'un protocole de balayage et/ou le paramétrage de l'unité d'acquisition d'images comprenant l'utilisation du des données d'image de la structure spécifique du marqueur lorsque l'unité d'acquisition d'image est l'unité MRI.

10. Système d'imagerie selon la revendication 9, dans lequel l'unité de traitement est configurée pour commander l'unité d'acquisition d'images pour acquérir des données d'image d'une région d'intérêt du patient.

11. Système d'imagerie selon la revendication 10, dans lequel l'unité de traitement est configurée pour supprimer ou réduire des données d'image du marqueur des données d'image de la région d'intérêt du patient.

12. Système d'imagerie selon la revendication 11, dans lequel l'unité de traitement est configurée pour utiliser des informations préalables relatives à la structure spécifique du marqueur pour supprimer ou réduire les données d'image du marqueur des données d'image de la région d'intérêt.

13. Procédé (200) d'imagerie d'un patient avec un système d'imagerie, comprenant:
- positionnement (210) d'un patient au moins partiellement dans une pluralité d'unités d'acquisition d'images du système d'imagerie, dans lequel un marqueur de patient selon l'une quelconque des revendications 1 à 9 est placé sur ou dans le patient, et dans lequel la pluralité d'unités d'acquisition d'images comprend une unité de radiographie ou une unité d'MRI;
- acquérir (220) des données d'image du patient, les données d'image du patient comprenant des données d'image de la structure spécifique du marqueur;
- identifier (230) le patient par une unité de traitement, l'identification comprenant l'utilisation des données d'image de la structure spécifique du marqueur lorsque l'unité d'acquisition d'image est l'unité de radiographie et l'identification du patient par l'unité de traitement, l'identification comprenant l'exploitation des données d'images de la structure spécifique du marqueur lorsque l'unité d'acquisition d'images est l'unité MRI; et
- définir (240) au moins un paramètre relatif à un examen du patient comprenant le réglage (250) d'un protocole de balayage et/ou le réglage des paramètres de l'unité d'acquisition d'imagerie, la définition comprenant l'utilisation des données d'image de la structure spécifique du marqueur lorsque l'unité d'acquisition d'images est l'unité de radiographie et la définition d'au moins un paramètre relatif à un examen du patient comprenant le réglage d'un protocole de balayage et/ou le paramétrage de l'unité d'acquisition d'images, la définition comprenant l'utilisation des données d'image de la structure spécifique du marqueur lorsque l'unité d'acquisition d'images est l'unité MRI.
